# EUROPEAN PATENT SPECIFICATION

(11) **EP 3 998 038 B1**
(45) Date of publication and mention of the grant of the patent: **16.10.2024**
(21) Application number: 20837509.7
(22) Date of filing: 11.05.2020
(51) Int. Cl.: A61B 6/00, A61B 6/03, A61B 34/10, A61B 5/00, A61B 6/12, A61B 17/56, A61B 90/00, A61B 6/46, A61B 6/50, A61B 34/00, G16H 20/40, G16H 30/40, G16H 50/50, A61C 1/08, A61B 6/51, A61C 8/00

(54) **MULTIPLE BONE DENSITY DISPLAYING METHOD FOR ESTABLISHING IMPLANT PROCEDURE PLAN, AND IMAGE PROCESSING DEVICE THEREFOR**
VERFAHREN ZUR ANZEIGE VON MEHRFACHER KNOCHENDICHTE ZUR ERSTELLUNG EINES IMPLANTATIONSVORGANGSPLANS UND BILDVERARBEITUNGSVORRICHTUNG DAFÜR
PROCÉDÉ D'AFFICHAGE DE DENSITÉ OSSEUSE MULTIPLE POUR ÉTABLIR UN PLAN DE PROCÉDURE D'IMPLANT ET DISPOSITIF DE TRAITEMENT D'IMAGE ASSOCIÉ

(30) Priority: 11.07.2019 KR 20190083834
(43) Date of publication of application: 18.05.2022
(73) Proprietor: Osstem Implant Co., Ltd., Gangseo-gu Seoul 07789 (KR)
(72) Inventor: CHOI, Kyoo Ok, Seoul 07789 (KR); LEE, Do Hyun, Seoul 06968 (KR); SHIN, Ji Hye, Seoul 08097 (KR)
(74) Representative: Modiano, Gabriella Diana
(86) International application number: PCT/KR2020/006139
(87) International publication number: WO 2021/006472

(56) References cited:
- WO-A1-2018/066764
- CA-A1- 2 158 033
- KR-A- 20140 111 902
- KR-A- 20180 038 319
- KR-A- 20180 038 319
- KR-A- 20190 018 913
- KR-B1- 101 212 556
- KR-B1- 101 212 556
- US-A1- 2011 256 508
- US-A1- 2013 196 280
- US-A1- 2020 046 473
- SUGIURA TSUTOMU, YAMAMOTO KAZUHIKO, HORITA SATOSHI, MURAKAMI KAZUHIRO, TSUTSUMI SADAMI, KIRITA TADAAKI: "The effects of bone density and crestal cortical bone thickness on micromotion and peri-implant bone strain distribution in an immediately loaded implant: a nonlinear finite element analysis", JOURNAL OF PERIODONTAL & IMPLANT SCIENCE, vol. 46, no. 3, 1 January 2016 (2016-01-01), pages 152, XP055785228, ISSN: 2093-2278, DOI: 10.5051/jpis.2016.46.3.152

## Description

### Technical Field

The following description relates to a dental image processing technology, and more particularly, to a technology for providing a user interface for establishing an implant procedure plan, and image processing therefor.

### Background Art

Before an actual doctor performs an implant procedure, an implant procedure plan is established in advance through a virtual simulation using an implant simulation program. For example, a design process is performed in which an artificial tooth suitable for a patient is selected, and the artificial tooth is virtually placed at a target tooth position. The implant procedure plan includes determination of a position and type of an implant structure including a fixture for each target tooth to be operated. In order to determine the position and type of the implant structure, and to establish a procedure plan such as a drilling procedure or the like, it is required to identify a bone density of an implant site.

For example, systems and methods for identifying and displaying a bone density of an implant site are disclosed in prior art documents WO 2018/066764 A1, US 2020/046473 A1, US 2013/196280 A1, CA 2,158,033 A1, and US 2011/256508 A1.

### Disclosure of the Invention

### Technical Goals

An aspect provides a multiple bone density displaying method for allowing a user to efficiently identify a bone density of an implant site when an implant procedure plan is established and an image processing device therefor.

### Technical Solutions

According to an aspect, there is provided a multiple bone density displaying method including generating a virtual bone density display area with respect to a placed fixture in a dental image, and overlaying the virtual bone density display area at a position of the fixture, during establishment of an implant procedure plan, analyzing a bone density corresponding to the bone density display area, and displaying the analyzed bone density as color information in the bone density display area.

The generating of the virtual bone density display area with respect to the placed fixture in the dental image, and the overlaying of the virtual bone density display area at the position of the fixture may include generating the bone density display area spaced apart by a preset interval from a boundary line of the fixture and disposing the bone density display area on the fixture in a translucent state, and the preset interval of the bone density display area may be changeable by a user.

The displaying of the analyzed bone density as the color information in the bone density display area may include calculating an average bone density value of the bone density display area and displaying the calculated average bone density value as the color information.

The displaying of the analyzed bone density as the color information in the bone density display area may include classifying a bone density grade into a hard bone, a normal bone, and a soft bone according to bone quality, and displaying the bone density in a color corresponding to the classified bone density grade in the bone density display area.

The displaying of the analyzed bone density as the color information in the bone density display area may include receiving a user operation signal for entering a multiple bone density display mode, generating a plurality of consecutive cross-sectional images including a preset bone density display area in response to the received user operation signal, and providing consecutive bone density cross-sections by displaying the bone density as the color information in the bone density display area in respective consecutive cross-sectional images.

The multiple bone density displaying method may further include receiving a user operation signal for selecting a predetermined user setting area for identifying a bone density in the dental image, analyzing a bone density corresponding to a user setting area selected by a user in response to the received user operation signal, and displaying the analyzed bone density as the color information in the user setting area.

The multiple bone density displaying method may further include generating a plurality of consecutive cross-sectional images including the user setting area selected by the user, and providing consecutive bone density cross-sections by displaying the bone density as the color information in the user setting area in respective consecutive cross-sectional images.

The multiple bone density displaying method may further include providing an interface for selecting a bone density mode, and displaying the bone density in the form of an average value or a color map in response to selection of the bone density mode by a user operation signal.

The multiple bone density displaying method may further include providing a user interface for selecting a bone density view mode and displaying the bone density in the form of one of an axial view, a coronal view, and a sagittal view in response to selection of the bone density view mode by a user operation signal.

According to another aspect, there is provided an image processing device including an output unit configured to overlay, with respect to a placed fixture in a dental image, a virtual bone density display area at a position of the fixture, and display a bone density as color information in the bone density display area, when a bone density display mode is entered, an input unit configured to receive a user operation signal, and a controller configured to generate a bone density display area while performing the bone density display mode in response to the received user operation signal, analyze a bone density corresponding to a preset bone density display area, and then configure and provide, to the output unit, a screen for displaying the analyzed bone density as color information in the bone density display area.

The output unit may be configured to dispose the bone density display area spaced apart by a preset interval from a boundary line of the fixture on the fixture in a translucent state, and the preset interval of the bone density display area may be changeable by a user.

The controller may be configured to calculate an average bone density value of the bone density display area and configure a screen for displaying the calculated average bone density value as the color information.

The controller may be configured to classify a bone density grade into a hard bone, a normal bone, and a soft bone according to bone quality, and configure a screen for displaying the bone density in a color corresponding to the classified bone density grade in the bone density display area.

The input unit may be configured to receive a user operation signal for entering a multiple bone density display mode, and the controller may be configured to generate a plurality of consecutive cross-sectional images including a preset bone density display area in response to the received user operation signal, and provide consecutive bone density cross-sections by configuring a screen for displaying the bone density as the color information in the bone density display area in respective consecutive cross-sectional images, when the user operation signal for entering the multiple bone density display mode is received through the input unit.

The input unit may be configured to receive a user operation signal for selecting a predetermined user setting area for identifying the bone density in the dental image, and the controller may be configured to analyze a bone density corresponding to a user setting area selected by a user in response to the received user operation signal and configure a screen for displaying the analyzed bone density as the color information in the user setting area.

The controller may be configured to generate a plurality of consecutive cross-sectional images including the user setting area selected by the user and provide consecutive bone density cross-sections by configuring a screen for displaying the bone density as the color information in the user setting area in respective consecutive cross-sectional images.

The output unit may be configured to provide an interface for selecting a bone density mode and display the bone density in the form of an average value or a color map in response to selection of the bone density mode by the user operation signal.

The output unit may be configured to provide an interface for selecting a bone density view mode and display the bone density in the form of one of an axial view, a coronal view, and a sagittal view in response to selection of the bone density view mode by the user operation signal.

### Advantageous Effects

According to a multiple bone density displaying method for establishing an implant procedure plan and an image processing device therefor according to aspects, there is provided a user interface for allowing to efficiently and accurately identify a bone density of an implant site required to establish a treatment plan, such as determination of a position and type of a fixture in a dental image, a drilling procedure, or the like. Accordingly, it is possible to more conveniently establish an implant procedure plan by making it easier for a user to visually observe the bone density.

In this case, the bone density may be consecutively observed on the fixture by displaying the bone density through a plurality of vertical cross-sectional images. In addition, the user may easily establish a placement plan by displaying a color corresponding to an average bone density value in a bone density display area.

When an area where the user desires to observe is directly set, the bone density is displayed through a plurality of vertical cross-sectional images corresponding to a user setting area, thereby identifying a consecutive cross-sectional bone density. Accordingly, the user may consecutively observe the bone density with respect to an area where the user desires to observe. In addition, the user may easily establish a placement plan by displaying a color corresponding to an average bone density value in a user display area.

Furthermore, the user may easily and conveniently identify a bone density for a consecutive part or another part.

### Brief Description of Drawings

FIG. 1 is a diagram illustrating a configuration of an image processing device according to an example embodiment.
FIG. 2 is a diagram illustrating a general bone density display mode screen.
FIG. 3 is a diagram illustrating an example of measuring a bone density of a general cross-sectional image.
FIG. 4 is a diagram illustrating a screen for entering a bone density display mode according to an example embodiment.
FIG. 5 is a diagram illustrating an example of a user operation for entering an automatic bone density display mode screen and a multiple bone density display mode according to an example embodiment.
FIG. 6 is a diagram illustrating a multiple bone density display mode screen according to an example embodiment.
FIG. 7 is a diagram illustrating a multiple bone density display mode screen according to another example embodiment.
FIG. 8 is a diagram illustrating a manual bone density display mode entry screen according to an example embodiment.
FIG. 9 is a diagram illustrating a manual bone density display mode screen according to an example embodiment.
FIG. 10 is a diagram illustrating a range of a bone density display area according to an example embodiment.
FIG. 11 is a diagram illustrating a flow of a multiple bone density displaying method for establishing an implant procedure plan according to an example embodiment.

### Best Mode for Carrying Out the Invention

The aspects and features of the present invention and methods for achieving the aspects and features will be apparent by referring to the example embodiments to be described in detail with reference to the accompanying drawings. However, the present invention is not limited to the example embodiments disclosed hereinafter, but can be implemented in various different forms. The example embodiments are merely provided so that the present invention is thorough and complete, and fully conveys the scope of the present invention to those skilled in the art, and the present invention is only defined within the scope of the appended claims. Throughout the entire specification, the same or like reference numerals designate the same or like elements.

In describing the example embodiments, a detailed description of related known configurations or functions incorporated herein will be omitted when it is determined that the detailed description thereof may unnecessarily obscure the subject matter of the present invention. The terms which will be described below are terms defined in consideration of the functions in the present invention, and may be different according to users, intentions of the users, or customs. Therefore, the definitions of the terms should be made based on the contents throughout the specification.

Combinations of blocks in the accompanying block diagrams or steps in the accompanying flowcharts can be executed by computer program instructions (execution engine), and the computer program instructions can be mounted in a processor of a general-use computer, special-use computer or other programmable data processing equipment. Thus, the instructions executed through the processor of the computer or other programmable data processing equipment generate units for performing functions described in the respective blocks of the block diagrams or the respective steps of the flowcharts.

The computer program instructions can be stored in a computer usable or readable memory oriented to a computer or other programmable data processing equipment, in order to implement functions in a specific method. Thus, the instructions stored in the computer usable or readable memory can be used to manufacture products including instruction units for performing the functions described in the respective blocks of the block diagrams or the respective steps of the flowcharts.

In addition, the computer program instructions can be mounted in the computer or other programmable data processing equipment. Therefore, instructions which generate processes by performing a series of operation steps on the computer or other programmable data processing equipment and operate the computer or other programmable data processing equipment can provide steps for executing the functions described in the respective blocks of the block diagrams and the respective steps of the flowcharts.

Each of the blocks or steps may indicate a part of a module, segment or code including one or more executable instructions for executing specific logical functions. In some substitutions, the functions described in the blocks or steps can be performed out of sequence. For example, two blocks or steps can be operated or performed substantially at the same time, and the blocks or steps can be operated or performed in a reverse order of the corresponding function.

Hereinafter, the example embodiments will be described in detail with reference to the accompanying drawings. However, the example embodiments may be modified in various different forms, and the scope of the present invention is not limited to the example embodiments described below. The example embodiments are provided to more completely describe the present invention to those skilled in the art.

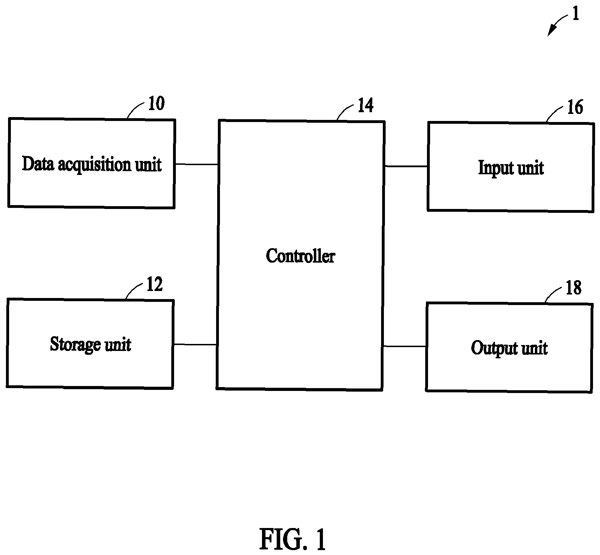

FIG. 1 is a diagram illustrating a configuration of an image processing device according to an example embodiment.

An image processing device 1 is an electronic device capable of executing a dental image processing program. The electronic device includes a computer, a notebook computer, a laptop computer, a tablet PC, a smart phone, a mobile phone, a personal media player (PMP), a personal digital assistant (PDA), and the like. The dental image processing program includes an implant simulation program, a guide design program, a scan program, a CAD program, and the like, and an implant operation plan may be placed therethrough. In addition, the dental image processing program may be applied to a program for processing a general medical image other than a program for dental implant surgery. The implant simulation program is a program for designing a position and direction of an implant structure while performing a simulation of placing a virtual implant object on a dental image.

Hereinafter, for ease of description, a guide design program for dental implant surgery will be described as an example. However, when image processing is possible, it is also applicable to other programs in the same manner.

A guide design process for dental implant surgery using a program includes processes of registering a surgical patient, acquiring CT data and oral model data of the registered patient, matching the CT data and the oral model data, generating an arch line from the matched image data and generating a panoramic image using the arch line, determining a position and size of a crown model in the oral model data of the patient, determining a position of an implant structure including a fixture in the CT data of the patient, designing a guide shape, and outputting a final guide.

The present invention relates to a technology for providing a user interface for allowing a user to efficiently and accurately identify a bone density when an implant procedure plan is established.

Referring to FIG. 1, the image processing device 1 according to an example embodiment includes a data acquisition unit 10, a storage unit 12, a controller 14, an input unit 16, and an output unit 18.

The data acquisition unit 10 acquires basic image data of the patient. The basic image data includes X-ray data, CT data, oral model data, and the like. The data acquisition unit 10 may execute the basic image data on a program or load data stored in a web page and a server. For placement of an implant, the CT data, the oral model data, and the like may be required as basic data.

The oral model data may be obtained by scanning a plaster model generated by imitating the patient's oral cavity with a 3D scanner, or by scanning the inside of the patient's oral cavity with a 3D intra-oral scanner. The CT data may be obtained by generating tomographic images of the patient's head using computed tomography (CT), segmenting a boundary of a tooth part in each tomographic image, and then combining the images into one. The acquired oral model data and CT data may be stored in the storage unit 12. The CT data may be provided in various cross-sectional image forms such as a sagittal view image, a coronal view image, and an axial view image, and may be provided in a 3D image form.

The storage unit 12 stores information required to perform an operation of the image processing device 1 and information generated according to the operation performed and provides the information when requested by the controller 14. The storage unit 12 may store a fixture library including various fixture models and may provide the fixture library when requested by the controller 14. The storage unit 12 according to an example embodiment stores an operation content including data of a guide object that is generated or changed for each operation step when a guide design operation is performed.

The controller 14 executes an image processing program. In particular, when an implant structure for an implant procedure is placed, a bone density display mode is performed in response to a user operation signal inputted from the input unit 16. In this case, a bone density is analyzed, and screen information for displaying the analyzed bone density as color information in a preset bone density display area or a user display area set by a user is configured, and then the screen information is provided to the output unit 18. Analysis of the bone density may include a process of calculating a Hounsfield unit (hereinafter referred to as "HU") value of a corresponding area to calculate a bone density according to the HU value.

The controller 14 may calculate an average bone density value of the bone density display area or the user display area, configure a screen for displaying the calculated average bone density value as color information, and provide the screen to the output unit 18. The average bone density value may be obtained by calculating an average HU value.

The controller 14 may classify a bone density grade into a hard bone, a normal bone, and a soft bone according to bone quality, and configure a screen for displaying the bone density in a color corresponding to the classified bone density grade in the bone density display area or the user display area.

The controller 14 controls the bone density to be displayed with respect to the fixture through a plurality of vertical cross-sectional images, so that the user may consecutively observes the bone density with respect to the fixture. For example, when a user operation signal for entering a multiple bone density display mode is inputted through the input unit 16, the controller 14 includes a plurality of consecutive cross-sectional images including a preset bone density display area in response to the inputted user operation signal. Then, a screen for displaying the bone density as color information is configured in the bone density display area in respective consecutive cross-sectional images, and then provided to the output unit 18. Accordingly, it is possible to provide consecutive bone density cross-sections with respect to the fixture.

The controller 14 controls to display the bone density through the plurality of vertical cross-sectional images with respect to a site the user wishes to observe, so that the user may consecutively observe the bone density with respect to the desired site. For example, when a user setting area desired by the user is inputted through the input unit 16, the controller 14 analyzes the bone density corresponding to the user setting area selected by the user, and configures a screen for displaying the analyzed bone density as color information in the user setting area, and then provides the screen to the output unit 18. Accordingly, it is possible to provide consecutive bone density cross-sections with respect to an area desired by the user.

The output unit 18 displays a program screen including pieces of information generated by the controller 14. The output unit 18 according to an example embodiment displays a user interface for entering an automatic bone density display mode (bone density (fixture)) or a manual bone density display mode (bone density (manual)) on the screen. When a predetermined bone density display mode is entered, a user interface for selecting a bone density mode, selecting a view mode, and the like may be displayed on the screen. In response to selection of the bone density mode, the bone density may be displayed in the form of an average value or a color map. In response to selection of the view mode, the bone density may be displayed in the form of one of an axial view, a coronal view, and a sagittal view. Furthermore, in response to selection of a multi-mode, the bone density may be displayed through a plurality of consecutive cross-sectional images.

When the automatic bone density display mode (bone density (fixture)) is entered, the output unit 18 according to an example embodiment overlays a virtual bone density display area at a position of the fixture with respect to a placed fixture in a dental image and displays the bone density as color information in the bone density display area. The output unit 18 may dispose the bone density display area spaced apart by a preset interval from a boundary line of the fixture on the fixture in a translucent state. The preset interval of the bone density display area may be changeable by the user. When the manual bone density display mode (bone density (manual)) is entered, the bone density may be displayed as the color information in the user display area selected by the user. In this case, the bone density may be displayed in the user display area in the plurality of consecutive cross-sectional images.

The input unit 16 receives the user operation signal. The input unit 16 according to an example embodiment receives a selection signal for a user interface for entering the automatic bone density display mode (bone density (fixture)) and manual bone density display mode (bone density (manual)), a selection signal for a user interface for selecting a bone density mode, a view mode, and the like when a predetermined bone density display mode is entered, and a selection signal for entering a multi-mode, and the like. The input unit 16 may receive the user operation signal for selecting the user display area for observing the bone density in the dental image displayed on the screen through the output unit 18.

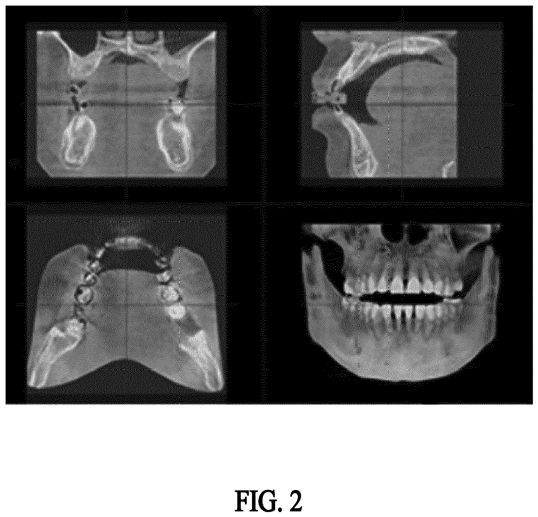

FIG. 2 is a diagram illustrating a general bone density display mode screen.

Referring to FIG. 2, the image processing device may execute implant simulation on a dental image so as to establish a plan for implant placement surgery of a patient. The dental image may be a CT image, and the CT image may be a 2D cross-sectional image, a 3D image, or the like. In order to efficiently place an implant structure, a function of identifying a bone density may be provided. When a general bone density identifying function is used, as illustrated in FIG. 2, the bone density is displayed in color on the entire screen according to an HU value of a dental image screen observed by a user. In this method, a bone density is displayed on the entire screen (2D image) to be diagnosed, so that the bone density is displayed even in a part where the user does not desire, and thus unnecessary information is excessively provided. Accordingly, a degree of fatigue may be increased during treatment. In addition, the bone density may only be observed on the screen currently viewed by the user, and thus additional operation is required when the user desires to identify a consecutive portion or another portion. Furthermore, since the bone density is only displayed in color according to the HU value, it is not possible to obtain display information on an average state of a bone density of a site where the implant structure is to be placed.

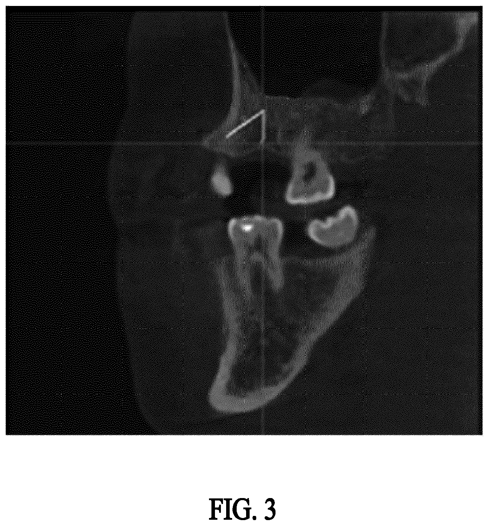

FIG. 3 is a diagram illustrating an example of measuring a bone density of a general cross-sectional image.

Referring to FIG. 3, in general, in order to identify a bone density of a part where an implant structure is to be placed, it is required to use an additional tool for measuring the bone density, and it is possible to make a measurement only in a cross-sectional image that is currently being observed, and thus it is difficult to observe a state of a three-dimensionally placed bone. Accordingly, there is a problem in that a user is not able to easily and conveniently observe and measure a desired part.

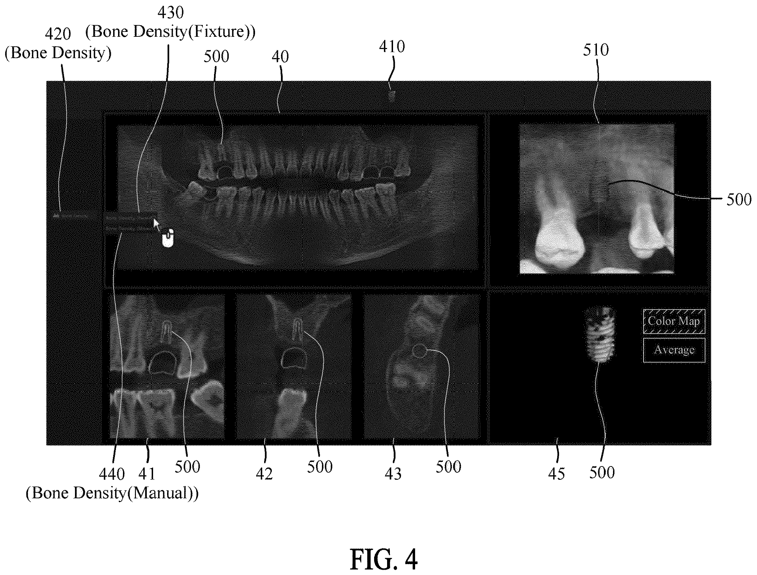

FIG. 4 is a diagram illustrating a screen for entering a bone density display mode according to an example embodiment.

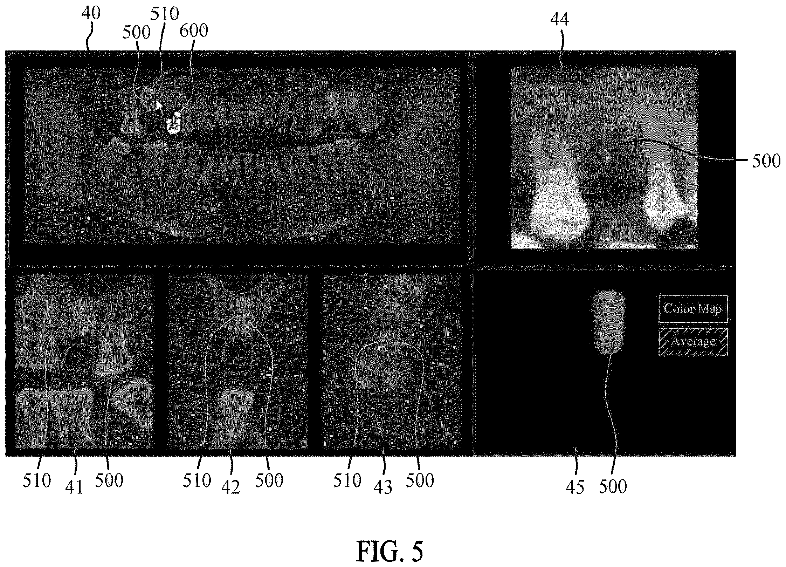

Referring to FIGS. 1 and 4, the image processing device 1 provides a dental image to establish a fixture placement plan through an image processing program. The dental image includes a panoramic image 40, CT cross-sectional images 41, 42, and 43, a 3D image 44, and fixture information 45. A user may automatically or manually place a virtual implant structure in the dental image. For example, the user places a predetermined fixture 500 in the dental image by selecting an implant module 410 through a click operation and the like. When the fixture is placed, as illustrated in FIG. 4, the virtual fixture 500 may be generated in each of the panoramic image 40, the sagittal view cross-sectional image 41, the coronal view cross-sectional image 42, the axial view cross-sectional image 43, and the 3D image 44. The fixture information 45 includes a tooth number in which a fixture is placed, a fixture manufacturer, a system, a diameter, a length, and a bone density image of the fixture. As illustrated in FIG. 4, the bone density image of the fixture may be displayed in multiple colors in the form of a color map, and may be displayed in a single color in the form of an average value by calculating an average bone density value. When displayed in multiple colors, a bone density grade may be classified into a hard bone, a normal bone and a soft bone according to bone quality, and the bone density of the fixture may be displayed in a color corresponding to the classified bone density grade. The bone density image of the fixture displayed in the form of an average value will be described later with reference to FIG. 5.

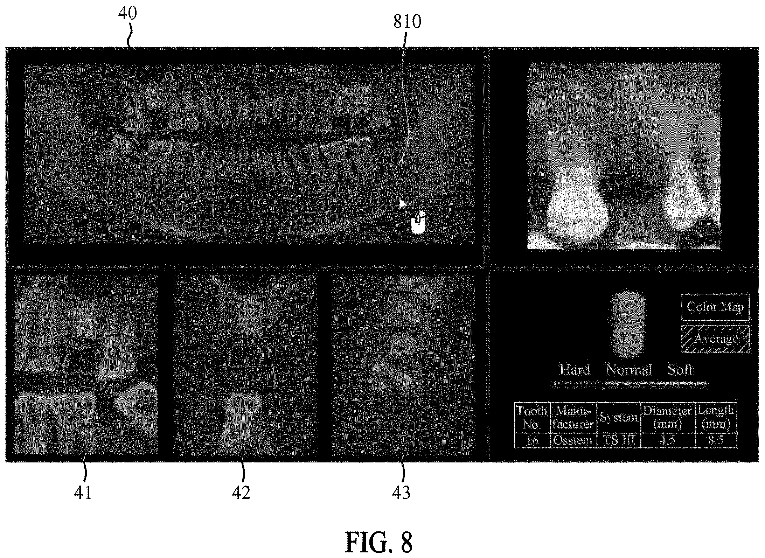

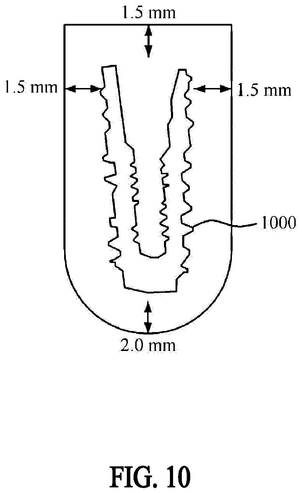

The image processing device 1 provides a bone density display mode function so that the user places the fixture 500 while identifying the bone density with respect to the fixture 500. To this end, when the user selects a bone density tool 420, a bone density (fixture) tool 430 and a bone density (manual) tool 440 are displayed on the screen. In this case, the user may enter an automatic bone density display mode by selecting the bone density (fixture) tool 430 through a click operation and the like. Then, a preset bone density display area is overlaid on the fixture 500 with respect to the fixture. A range of the preset bone density display area will be described later with reference to FIG. 10. As another example, the user may enter a manual bone density display mode by selecting the bone density (manual) tool 440 through a click operation and the like. In this case, the user may directly select a user display area for identifying the bone density. An example embodiment thereof will be described later with reference to FIG. 8.

FIG. 5 is a diagram illustrating an example of a user operation for entering an automatic bone density display mode screen and a multiple bone density display mode according to an example embodiment.

Referring to FIGS. 1 and 5, when the image processing device 1 enters the automatic bone density display mode through operation of the bone density (fixture) tool, and the like, a virtual bone density display area 510 is generated with respect to a placed fixture in a dental image and is overlaid at a position of the fixture 500. In this case, a bone density corresponding to the bone density display area 510 is displayed as color information. In FIG. 5, the bone density display area 510 has an upper surface with a round square shape, but the shape is not limited thereto. For example, the shape may be deformed into various shapes such as a rectangle, a cuboid, and the like. In addition, the shape may vary depending on a type of dental image. As illustrated in FIG. 5, the axial view cross-sectional image 43 may be displayed on a screen in the form of a circle.

The bone density display area 510 is a user interface displayed on the screen so that a user intuitively identifies a bone density through color. As the bone density display area 510 is disposed at a position of the fixture 500 in a translucent form, the shape of the fixture 500 may be identified together. A bone density of the bone density display area 510 may be displayed in multiple colors in the form of a color map and may be displayed in a single color in the form of an average bone density value, as illustrated in FIG. 5. When displayed in multiple colors, a bone density grade may be classified into a hard bone, a normal bone, and a soft bone according to bone quality, and the bone density may be displayed in a color corresponding to the classified bone density grade.

The image processing device 1 according to an example embodiment provides a multiple bone density display mode function of displaying a bone density through a plurality of consecutive vertical cross-sectional images so that the bone density is consecutively observed with respect to a fixture. To this end, as illustrated in FIG. 5, a user may enter the multiple bone density display mode by performing a double-click operation on the predetermined fixture 500 or the bone density display area 510 to be observed in the panoramic image 40. When the user desires to view consecutive cross-sectional bone density images of another fixture, the consecutive cross-sectional bone density images of the corresponding fixture may be displayed through a user operation such as a double-click operation performed on the fixture.

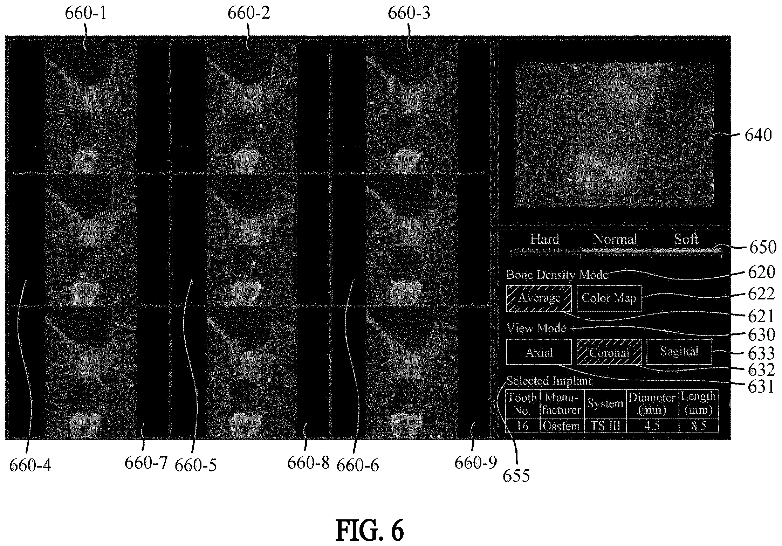

FIG. 6 is a diagram illustrating a multiple bone density display mode screen according to an example embodiment.

Referring to FIG. 6, in response to a user operation signal for entering the multiple bone density display mode, a plurality of consecutive cross-sectional images 610-1, 610-2, ..., and 610-9 including a preset bone density display area are generated and displayed. In this case, as a bone density is displayed as color information in each of bone density display areas in the plurality of consecutive cross-sectional images 610-1, 610-2, ..., and 610-9, consecutive bone density cross-sections are provided. The plurality of consecutive cross-sectional images 610-1, 610-2, ..., and 610-9 may be one of a sagittal view cross-sectional image, a coronal view cross-sectional image, and an axial view cross-sectional image. To this end, one of an axial view mode 631, a coronal view mode 632, a sagittal view mode 633 may be selected by providing a view mode interface 630 on a screen. The number of respective cross-sectional images is preset, and the number may be modified by a user. In FIG. 6, nine cross-sectional images are displayed. A position of each cross-sectional image may be displayed on a scout screen 640. For example, as illustrated in FIG. 6, positions of nine cross-sectional images may be identified with respect to a fixture along an arch line.

The user may select a bone density mode through a bone density mode interface 620. The bone density mode includes an average bone density value mode 621 and a color map mode 622. Through implant information (selected implant) 655, it is possible to identify a tooth number in which a placed implant structure is placed, a manufacturer, a system, a length, a diameter, and the like. Through a user operation such as a mouse scroll or the like, it is possible to move to a predetermined cross-sectional image among the plurality of consecutive cross-sectional images 610-1, 610-2, ..., and 610-9.

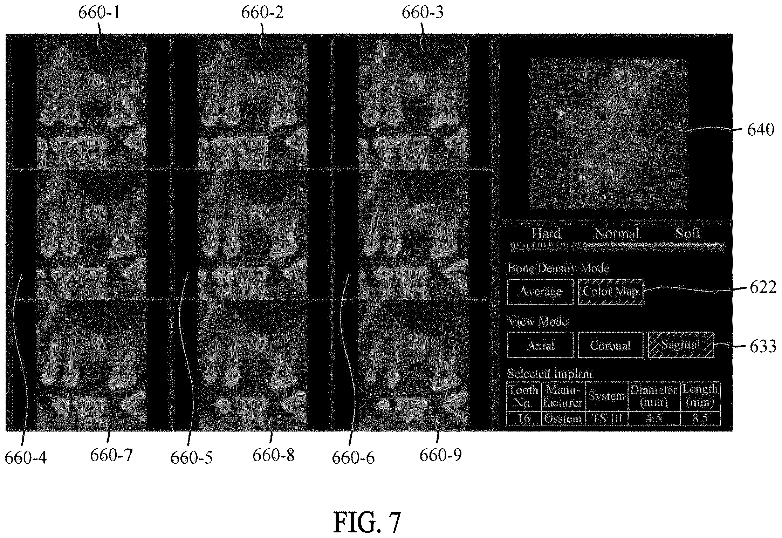

FIG. 7 is a diagram illustrating a multiple bone density display mode screen according to another example embodiment.

When the multiple bone density display mode screen of FIG. 6 corresponds to a case in which a bone density mode is the average bone density value mode 621, and a view mode is the coronal view mode 632, the multiple bone density display mode screen of FIG. 7 corresponds to a case in which a bone density mode is the color map mode 622, and a view mode is the sagittal view mode 633. In this case, on the scout screen 640, positions of the nine cross-sectional images may be identified with respect to a fixture along an axis of a sagittal view.

Referring to FIGS. 6 and 7, by displaying a bone density through a plurality of vertical cross-sectional images with respect to a fixture, the bone density may be consecutively observed with respect to the fixture. In addition, by displaying the bone density in a color corresponding to an average bone density value in a bone density display area, a user may easily establish a placement plan.

FIG. 8 is a diagram illustrating a manual bone density display mode entry screen according to an example embodiment.

Referring to FIG. 8, a user may enter the manual bone density display mode by selecting the bone density (manual) tool (440 of FIG. 4) through a click operation and the like. In this case, the user may directly select a bone density display area for identifying a bone density.

When the manual bone density display mode is entered, the user selects a predetermined user setting area 810 for identifying a bone density in a dental image, for example, the panoramic image 40. As illustrated in FIG. 8, the user setting area may be selected through an operation clicking and dragging a mouse but is not limited thereto.

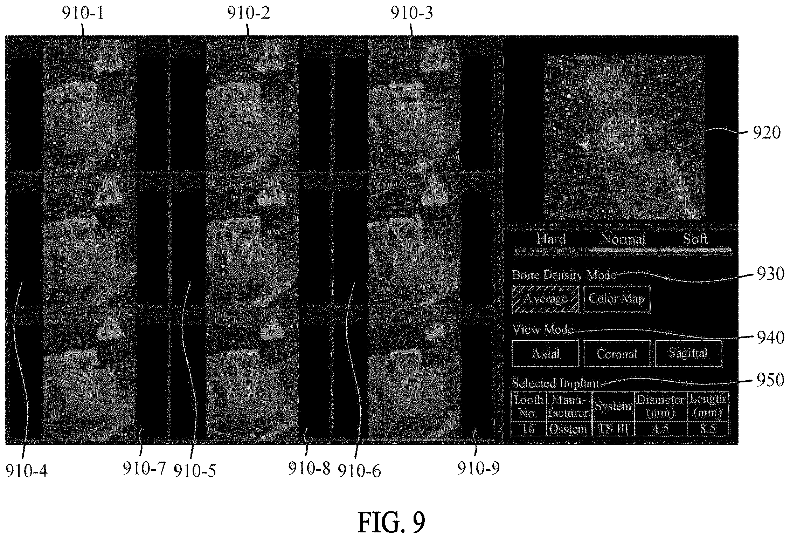

FIG. 9 is a diagram illustrating a manual bone density display mode screen according to an example embodiment.

Referring to FIG. 9, when the manual bone density display mode is entered, a bone density is displayed as color information in a user setting area. In this case, by generating a plurality of consecutive cross-sectional images 910-1, 910-2, ..., and 910-9 including the user setting area selected by a user and displaying the bone density as the color information in the user setting area in respective consecutive cross-sectional images 910-1, 910-2, ..., 910-9, consecutive bone density cross-sections may be provided. Even in the manual bone density display mode, a position of each cross-sectional image may be identified through a scout screen 920, a bone density mode may be selected through a bone density mode interface 930, and a view mode may be selected through a view mode interface 940. Implant information (selected implant) 950 displays information on a placed implant structure.

Referring to FIG. 9, when a user sets a desired area, a consecutive cross-sectional bone density may be identified by displaying the bone density through a plurality of vertical cross-sectional images corresponding to the user setting area. Accordingly, the user may consecutively observe the bone density with respect to an area where the user desires to observe. In addition, the user may easily establish a placement plan by displaying a color corresponding to an average bone density value in a user display area.

FIG. 10 is a diagram illustrating a range of a bone density display area according to an example embodiment.

Referring to FIG. 10, a bone density display area spaced apart from a boundary line of a fixture 1000 by a preset interval may be generated. As illustrated in FIG. 10, the preset interval may be 1.5 mm in an upward direction, 1.5 mm in each of left and right directions, and 2.0 mm in a downward direction. The preset interval of the bone density display area may be changeable by a user.

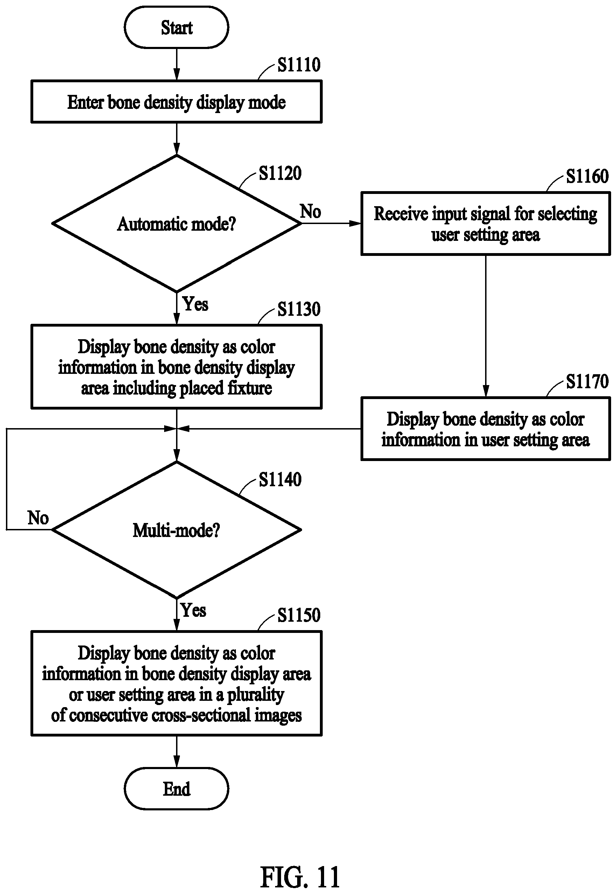

FIG. 11 is a diagram illustrating a flow of a multiple bone density displaying method for establishing an implant procedure plan according to an example embodiment.

Referring to FIGS. 1 and 11, the image processing device 1 enters a bone density display mode through an image processing program (S1110). For example, the image processing device 1 enters an automatic bone density display mode by receiving, from a user, an operation signal through a click operation performed on the bone density (fixture) tool 430 and the like so that the user places a fixture while identifying a bone density with respect to the fixture. As another example, the manual bone density display mode is entered by receiving, from the user, an operation signal through a click operation performed on the bone density (manual) tool 440.

Subsequently, the image processing device 1 determines whether it is in the automatic bone density display mode (S 1120), and in the automatic bone density display mode, the image processing device 1 generates a bone density display area including a placed fixture, overlays the bone density display area at a position of the fixture, analyzes a bone density corresponding to the bone density display area, and displays the analyzed bone density as color information in the bone density display area (S 1130). In this case, a bone density display area spaced apart by a preset interval from a boundary line of the fixture may be generated and disposed on the fixture in a translucent state. The preset interval of the bone density display area may be changeable by the user.

In step S1130 of displaying the bone density as the color information in the bone density display area, the image processing device 1 may calculate an average bone density value of the bone density display area, and then display the calculated average bone density value as the color information. As another example, after a bone density grade is classified into a hard bone, a normal bone, and a soft bone according to bone quality, the bone density may be displayed in a color corresponding to the classified bone density grade in the bone density display area.

Furthermore, the image processing device 1 may determine whether it is in a multi-mode (S 1140), and, in the multi-mode, the image processing device 1 may display the bone density as the color information in the bone density display area in a plurality of consecutive cross-sectional images (S 1150). To this end, a multiple bone density display mode may be entered through an double-click operation performed on the bone density display area where the user desires to observes in a panoramic image.

As another example, the image processing device 1 determines whether it is in the automatic bone density display mode (S 1120), and in the manual bone density display mode, the image processing device 1 receives a user operation signal for selecting a predetermined user setting area for identifying a bone density in a dental image. (S 1160). And, the image processing device 1 analyzes a bone density corresponding to the user setting area selected by the user in response to the received user operation signal, and then displays the analyzed bone density as the color information in the user setting area (S 1170). In this case, the image processing device 1 may determine whether it is in the multi-mode (S1140), and, in the multi-mode, the image processing device 1 may display the bone density as the color information in the user display area in the plurality of consecutive cross-sectional images (S 1150). In the manual bone density display mode, when the user setting area is selected, the user may enter the multi-mode immediately, and display the bone density as the color information in the user display area in the plurality of cross-sectional images.

While the present invention includes example embodiments, it will be apparent to one of ordinary skill in the art that various changes in form and details may be made in these example embodiments without departing from the scope of the claims. The example embodiments described herein are to be considered in a descriptive sense only, and not for purposes of limitation. Therefore, the scope of the present invention is defined not by the detailed description, but by the claims.

## Claims

1. A multiple bone density displaying method comprising:
generating a virtual bone density display area (510) with respect to a placed fixture (500) in a dental image, and overlaying the virtual bone density display area (510) at a position of the fixture (500), during establishment of an implant procedure plan;
analyzing a bone density corresponding to the bone density display area (510); and
displaying the analyzed bone density as color information in the bone density display area (510),
wherein the virtual bone density display area (510) is disposed at the position of the fixture (500) in a translucent form.

2. The multiple bone density displaying method of claim 1, wherein
the generating of the virtual bone density display area (510) with respect to the placed fixture (500) in the dental image, and the overlaying of the virtual bone density display area (510) at the position of the fixture (500) comprises generating the bone density display area (510) spaced apart by a preset interval from a boundary line of the fixture (500), and wherein further the bone density display area is disposed on the fixture in a translucent state, and
the preset interval of the bone density display area (510) is changeable by a user.

3. The multiple bone density displaying method of claim 1, wherein the displaying of the analyzed bone density as the color information in the bone density display area (510) comprises:
calculating an average bone density value of the bone density display area (510); and
displaying the calculated average bone density value as the color information.

4. The multiple bone density displaying method of claim 1, wherein the displaying of the analyzed bone density as the color information in the bone density display area (510) comprises:
classifying a bone density grade into a hard bone, a normal bone, and a soft bone according to bone quality; and
displaying the bone density in a color corresponding to the classified bone density grade in the bone density display area (510).

5. The multiple bone density displaying method of claim 1, wherein the displaying of the analyzed bone density as the color information in the bone density display area (510) comprises:
receiving a user operation signal for entering a multiple bone density display mode;
generating a plurality of consecutive cross-sectional images (610-1, 610-2, ..., 610-9) including a preset bone density display area in response to the received user operation signal; and
providing consecutive bone density cross-sections by displaying the bone density as the color information in the bone density display area (510) in respective consecutive cross-sectional images (610-1, 610-2, ..., 610-9).

6. The multiple bone density displaying method of claim 1, further comprising:
receiving a user operation signal for selecting a predetermined user setting area (810) for identifying a bone density in the dental image;
analyzing a bone density corresponding to a user setting area (810) selected by a user in response to the received user operation signal; and
displaying the analyzed bone density as the color information in the user setting area (810).

7. The multiple bone density displaying method of claim 6, further comprising:
generating a plurality of consecutive cross-sectional images (910-1, 910-2, ..., 910-9) including the user setting area (810) selected by the user; and
providing consecutive bone density cross-sections by displaying the bone density as the color information in the user setting area (810) in respective consecutive cross-sectional images (910-1, 910-2, ..., 910-9).

8. The multiple bone density displaying method of claim 1, further comprising:
providing an interface for selecting a bone density mode; and
displaying the bone density in the form of an average value or a color map in response to selection of the bone density mode by a user operation signal.

9. The multiple bone density displaying method of claim 1, further comprising:
providing a user interface for selecting a bone density view mode; and
displaying the bone density in the form of one of an axial view, a coronal view, and a sagittal view in response to selection of the bone density view mode by a user operation signal.

10. An image processing device (1) comprising:
an output unit (18) configured to overlay, with respect to a placed fixture (500) in a dental image, a virtual bone density display area (510) at a position of the fixture (500), and display a bone density as color information in the bone density display area (510), when a bone density display mode is entered;
an input unit (16) configured to receive a user operation signal; and
a controller (14) configured to generate a bone density display area (510) while performing the bone density display mode in response to the received user operation signal, analyze a bone density corresponding to a preset bone density display area, and then configure and provide, to the output unit (18), a screen for displaying the analyzed bone density as color information in the bone density display area (510),
wherein the virtual bone density display area (510) is disposed at the position of the fixture (500) in a translucent form.

11. The image processing device (1) of claim 10, wherein
the output unit (18) is further configured to dispose the bone density display area (510) spaced apart by a preset interval from a boundary line of the fixture (500) on the fixture in a translucent state,
the preset interval of the bone density display area (510) is changeable by a user.

12. The image processing device (1) of claim 10, wherein the controller (14) is configured to:
calculate an average bone density value of the bone density display area (510); and
configure a screen for displaying the calculated average bone density value as the color information.

13. The image processing device (1) of claim 10, wherein the controller (14) is configured to:
classify a bone density grade into a hard bone, a normal bone, and a soft bone according to bone quality; and
configure a screen for displaying the bone density in a color corresponding to the classified bone density grade in the bone density display area (510).

14. The image processing device (1) of claim 10, wherein
the input unit (16) is configured to receive a user operation signal for entering a multiple bone density display mode, and
the controller (14) is configured to generate a plurality of consecutive cross-sectional images (610-1, 610-2, ..., 610-9) including a preset bone density display area in response to the received user operation signal, and provide consecutive bone density cross-sections by configuring a screen for displaying the bone density as the color information in the bone density display area (510) in respective consecutive cross-sectional images (610-1, 610-2, ..., 610-9), when the user operation signal for entering the multiple bone density display mode is received through the input unit (16).

15. The image processing device (1) of claim 10, wherein
the input unit (16) is configured to receive a user operation signal for selecting a predetermined user setting area (810) for identifying the bone density in the dental image; and
the controller (14) is configured to analyze a bone density corresponding to a user setting area (810) selected by a user in response to the received user operation signal and configure a screen for displaying the analyzed bone density as the color information in the user setting area (810).

## Patentansprüche

1. Ein Mehrfach-Knochendichte-Darstellungsverfahren, das Folgendes umfasst:
das Erstellen eines virtuellen Knochendichte-Darstellungsbereichs (510) mit Bezug auf eine positionierte Verankerung (500) in einem Dentalbild und das Darüberlegen des virtuellen Knochendichte-Darstellungsbereichs (510) an einer Position der Verankerung (500) während der Erstellung eines Implantationsvorgangsplans;
das Analysieren einer Knochendichte entsprechend dem Knochendichte-Darstellungsbereich (510) und
das Darstellen der analysierten Knochendichte als Farbinformation im Knochendichte-Darstellungsbereich (510);
wobei der virtuelle Knochendichte-Darstellungsbereich (510) an der Position der Verankerung (500) in durchsichtiger Form angeordnet ist.

2. Das Mehrfach-Knochendichte-Darstellungsverfahren gemäß Anspruch 1, worin das Erstellen des virtuellen Knochendichte-Darstellungsbereichs (510) mit Bezug auf die positionierte Verankerung (500) im Dentalbild und das Darüberlegen des virtuellen Knochendichte-Darstellungsbereichs (510) an der Position der Verankerung (500) das Erstellen des Knochendichte-Darstellungsbereichs (510), in einem vordefinierten Abstand von einer Grenzlinie der Verankerung (500) beabstandet, umfassen; und wobei der Knochendichte-Darstellungsbereich weiter auf der Verankerung in einem durchsichtigen Zustand angeordnet ist, und
der vordefinierte Abstand des Knochendichte-Darstellungsbereichs (510) durch einen Benutzer veränderbar ist.

3. Das Mehrfach-Knochendichte-Darstellungsverfahren gemäß Anspruch 1, worin das Darstellen der analysierten Knochendichte als Farbinformation im Knochendichte-Darstellungsbereich (510) Folgendes umfasst:
das Berechnen eines durchschnittlichen Knochendichtewerts des Knochendichte-Darstellungsbereichs (510)
und
das Darstellen des berechneten durchschnittlichen Knochendichtewerts als Farbinformation.

4. Das Mehrfach-Knochendichte-Darstellungsverfahren gemäß Anspruch 1, worin das Darstellen der analysierten Knochendichte als Farbinformation im Knochendichte-Darstellungsbereich (510) Folgendes umfasst:
das Klassifizieren der Knochendichte in harte Knochen, normale Knochen und weiche Knochen, je nach Knochenqualität; und
das Darstellen der Knochendichte im Knochendichte-Darstellungsbereich (510) in einer Farbe, die der Klassifikation der Knochendichte entspricht.

5. Das Mehrfach-Knochendichte-Darstellungsverfahren gemäß Anspruch 1, worin das Darstellen der analysierten Knochendichte als Farbinformation im Knochendichte-Darstellungsbereich (510) Folgendes umfasst:
das Empfangen eines Nutzer-Betriebssignals zum Eintreten in einen Mehrfach-Knochendichte-Darstellungsmodus;
das Erstellen einer Vielzahl aufeinander folgender Querschnittsdarstellungen (610-1, 610-2, ..., 610-9), die einen vordefinierten Knochendichte-Darstellungsbereich einschließen, als Reaktion auf das empfangene Nutzer-Betriebssignal; und
das Erstellen aufeinander folgender Knochendichte-Querschnitte durch Darstellen der Knochendichte als Farbinformation im Knochendichte-Darstellungsbereich (510) in entsprechenden aufeinander folgenden Querschnittsdarstellungen (610-1, 610-2, ..., 610-9).

6. Das Mehrfach-Knochendichte-Darstellungsverfahren gemäß Anspruch 1, das weiter Folgendes umfasst:
das Empfangen eines Nutzer-Betriebssignals zum Auswählen eines vordefinierten Nutzer-Einstellungsbereichs (810) für die Bestimmung einer Knochendichte im Dentalbild;
das Analysieren einer Knochendichte entsprechend einem Nutzer-Einstellungsbereich (810), der von einem Benutzer ausgewählt wird, als Reaktion auf das empfangene Nutzer-Betriebssignal; und
das Darstellen der analysierten Knochendichte als Farbinformation im Nutzer-Einstellungsbereich (810).

7. Das Mehrfach-Knochendichte-Darstellungsverfahren gemäß Anspruch 6, das weiter Folgendes umfasst:
das Erstellen einer Vielzahl aufeinander folgender Querschnittsdarstellungen (910-1, 910-2, ..., 910-9), die den vom Benutzer gewählten Nutzer-Einstellungsbereich (810) einschließen, und
das Bereitstellen aufeinander folgender Knochendichte-Querschnitte durch Darstellen der Knochendichte als Farbinformation im Nutzer-Einstellungsbereich (810) in entsprechenden aufeinander folgenden Querschnittsdarstellungen (910-1, 910-2, ..., 910-9).

8. Das Mehrfach-Knochendichte-Darstellungsverfahren gemäß Anspruch 1, das weiter Folgendes umfasst:
das Bereitstellen einer Schnittstelle zum Auswählen eines Knochendichtemodus und
das Darstellen der Knochendichte in Form eines Durchschnittswerts oder einer Farbzuordnungstabelle als Reaktion auf die Auswahl des Knochendichtemodus durch ein Nutzer-Betriebssignal.

9. Das Mehrfach-Knochendichte-Darstellungsverfahren gemäß Anspruch 1, das weiter Folgendes umfasst:
das Bereitstellen einer Benutzerschnittstelle zum Auswählen eines Knochendichte-Ansichtsmodus und
das Darstellen der Knochendichte in Form entweder einer axialen Ansicht, einer koronalen Ansicht oder einer sagittalen Ansicht als Reaktion auf die Auswahl des Knochendichte-Ansichtsmodus durch ein Nutzer-Betriebssignal.

10. Eine Bildverarbeitungsvorrichtung (1), die Folgendes umfasst:
eine Ausgabeeinheit (18), konfiguriert, um, mit Bezug auf eine positionierte Verankerung (500) in einem Dentalbild, einen virtuellen Knochendichte-Darstellungsbereich (510) an einer Position der Verankerung (500) darüber zu legen und eine Knochendichte als Farbinformation im Knochendichte-Darstellungsbereich (510) darzustellen, wenn ein Knochendichte-Darstellungsmodus eingegeben wird;
eine Eingabeeinheit (16), konfiguriert, um ein Nutzer-Betriebssignal zu empfangen; und
eine Steuerung (14), konfiguriert, um einen Knochendichte-Darstellungsbereich (510) zu erstellen bei gleichzeitiger Ausführung des Knochendichte-Darstellungsmodus als Reaktion auf das empfangene Nutzer-Betriebssignal; eine Knochendichte entsprechend einem vordefinierten Knochendichte-Darstellungsbereich zu analysieren und anschließend eine Anzeige zur Darstellung der analysierten Knochendichte als Farbinformation im Knochendichte-Darstellungsbereich (510) zu konfigurieren und der Ausgabeeinheit (18) zur Verfügung zu stellen;
wobei der virtuelle Knochendichte-Darstellungsbereich (510) in der Position der Verankerung (500) in durchsichtiger Form angeordnet ist.

11. Die Bildverarbeitungsvorrichtung (1) gemäß Anspruch 10, wobei die Ausgabeeinheit (18) weiter konfiguriert ist, um den Knochendichte-Darstellungsbereich (510) in einem vordefinierten Abstand von einer Grenzlinie der Verankerung (500) beabstandet auf der Verankerung in einem durchsichtigen Zustand anzuordnen;
der vordefinierte Abstand des Knochendichte-Darstellungsbereichs (510) ist durch einen Benutzer veränderbar.

12. Die Bildverarbeitungsvorrichtung (1) gemäß Anspruch 10, wobei die Steuerung (14) konfiguriert ist, um
einen durchschnittlichen Knochendichtewert des Knochendichte-Darstellungsbereichs (510) zu berechnen und
eine Anzeige zu konfigurieren, um den berechneten durchschnittlichen Knochendichtewert als Farbinformation anzuzeigen.

13. Die Bildverarbeitungsvorrichtung (1) gemäß Anspruch 10, wobei die Steuerung (14) konfiguriert ist, um
die Knochendichte in harte Knochen, normale Knochen und weiche Knochen zu klassifizieren, je nach Knochenqualität; und
eine Anzeige zu konfigurieren, um die Knochendichte im Knochendichte-Darstellungsbereich (510) in einer Farbe anzuzeigen, die der Klassifikation der Knochendichte entspricht.

14. Die Bildverarbeitungsvorrichtung (1) gemäß Anspruch 10, wobei die Eingabeeinheit (16) konfiguriert ist, um ein Nutzer-Betriebssignal zur Eingabe eines Mehrfach-Knochendichte-Darstellungsmodus zu empfangen, und die Steuerung (14) konfiguriert ist, um eine Vielzahl aufeinander folgender Querschnittsdarstellungen (610-1, 610-2, ..., 610-9) zu erstellen, die einen vordefinierten Knochendichte-Darstellungsbereich einschließen, als Reaktion auf das empfangene Nutzer-Betriebssignal; und aufeinander folgende Knochendichte-Querschnitte bereitzustellen durch Konfigurieren einer Anzeige, um die Knochendichte als Farbinformation im Knochendichte-Darstellungsbereich (510) in entsprechenden aufeinander folgenden Querschnittsdarstellungen (610-1, 610-2, ..., 610-9) darzustellen, wenn das Nutzer-Betriebssignal zum Eingeben des Mehrfach-Knochendichte-Darstellungsmodus durch die Eingabeeinheit (16) empfangen wird.

15. Die Bildverarbeitungsvorrichtung (1) gemäß Anspruch 10, wobei
die Eingabeeinheit (16) konfiguriert ist, um ein Nutzer-Betriebssignal zu empfangen, um einen vordefinierten Nutzer-Einstellungsbereich (810) zur Bestimmung der Knochendichte im Dentalbild auszuwählen; und
die Steuerung (14) konfiguriert ist, um eine Knochendichte zu analysieren, die einem von einem Benutzer ausgewählten Nutzer-Einstellungsbereich (810) entspricht, als Reaktion auf das empfangene Nutzer-Betriebssignal, und eine Anzeige zur Darstellung der analysierten Knochendichte als Farbinformation im Nutzer-Einstellungsbereich (810) zu konfigurieren.

## Revendications

1. Procédé d'affichage de densité osseuse multiple comprenant les étapes suivantes :
générer une région d'affichage de densité osseuse virtuelle (510) par rapport à un dispositif de fixation posé (500) dans une image dentaire, et superposer la région d'affichage de densité osseuse virtuelle (510) sur une position du dispositif de fixation (500), pendant l'établissement d'un plan de procédure d'implantation ;
analyser une densité osseuse correspondant à la région d'affichage de densité osseuse (510) ; et
afficher la densité osseuse analysée sous forme d'information de couleur dans la région d'affichage de densité osseuse (510),
dans lequel la région d'affichage de densité osseuse virtuelle (510) est placée à la position du dispositif de fixation (500) sous une forme transparente.

2. Procédé d'affichage de densité osseuse multiple selon la revendication 1, dans lequel :
la génération de la région d'affichage de densité osseuse virtuelle (510) par rapport au dispositif de fixation posé (500) dans l'image dentaire, et la superposition de la région d'affichage de densité osseuse virtuelle (510) sur la position du dispositif de fixation (500) comprennent le fait de générer la région d'affichage de densité osseuse (510) espacée d'un intervalle préétabli par rapport à une ligne de délimitation du dispositif de fixation (500), et dans lequel en outre la région d'affichage de densité osseuse est placée sur le dispositif de fixation dans un état transparent, et
l'intervalle préétabli de la région d'affichage de densité osseuse (510) peut être modifié par un utilisateur.

3. Procédé d'affichage de densité osseuse multiple selon la revendication 1, dans lequel l'affichage de la densité osseuse analysée sous forme d'information de couleur dans la région d'affichage de densité osseuse (510) comprend les étapes suivantes :
calculer une valeur de densité osseuse moyenne de la région d'affichage de densité osseuse (510) ; et
afficher la valeur de densité osseuse moyenne calculée sous la forme de ladite information de couleur.

4. Procédé d'affichage de densité osseuse multiple selon la revendication 1, dans lequel l'affichage de la densité osseuse analysée sous forme d'information de couleur dans la région d'affichage de densité osseuse (510) comprend les étapes suivantes :
classer un niveau de densité osseuse en os dur, os normal et os mou selon la qualité osseuse ; et
afficher la densité osseuse dans une couleur correspondant au niveau de densité osseuse déterminé dans la région d'affichage de densité osseuse (510).

5. Procédé d'affichage de densité osseuse multiple selon la revendication 1, dans lequel l'affichage de la densité osseuse analysée sous forme d'information de couleur dans la région d'affichage de densité osseuse (510) comprend les étapes suivantes :
recevoir un signal de fonctionnement d'utilisateur pour entrer un mode d'affichage de densité osseuse multiple ;
générer une pluralité d'images en coupe consécutives (610-1, 610-2, ..., 610-9) comportant une région d'affichage de densité osseuse préétablie en réponse au signal de fonctionnement d'utilisateur reçu ; et
fournir des coupes de densité osseuse consécutives en affichant la densité osseuse sous la forme de l'information de couleur dans la région d'affichage de densité osseuse (510) dans les images en coupe consécutives respectives (610-1, 610-2, ..., 610-9).

6. Procédé d'affichage de densité osseuse multiple selon la revendication 1, comprenant en outre les étapes suivantes :
recevoir un signal de fonctionnement d'utilisateur pour sélectionner une région de réglage d'utilisateur prédéterminée (810) pour identifier une densité osseuse dans l'image dentaire ;
analyser une densité osseuse correspondant à une région de réglage d'utilisateur (810) sélectionnée par un utilisateur en réponse au signal de fonctionnement d'utilisateur reçu ; et
afficher la densité osseuse analysée sous la forme de l'information de couleur dans la région de réglage d'utilisateur (810).

7. Procédé d'affichage de densité osseuse multiple selon la revendication 6, comprenant en outre les étapes suivantes :
générer une pluralité d'images en coupe consécutives (910-1, 910-2, ..., 910-9) comportant la région de réglage d'utilisateur (810) sélectionnée par l'utilisateur ; et
fournir des coupes de densité osseuse consécutives en affichant la densité osseuse sous la forme de l'information de couleur dans la région de réglage d'utilisateur (810) dans les images en coupe consécutives respectives (910-1, 910-2, ..., 910-9).

8. Procédé d'affichage de densité osseuse multiple selon la revendication 1, comprenant en outre les étapes suivantes :
fournir une interface pour sélectionner un mode de densité osseuse ; et
afficher la densité osseuse sous la forme d'une valeur moyenne ou d'une carte en couleur en réponse à la sélection du mode de densité osseuse par un signal de fonctionnement d'utilisateur.

9. Procédé d'affichage de densité osseuse multiple selon la revendication 1, comprenant en outre les étapes suivantes :
fournir une interface utilisateur pour sélectionner un mode de visualisation de densité osseuse ; et
afficher la densité osseuse sous la forme d'une vue parmi une vue axiale, une vue coronaire et une vue sagittale en réponse à la sélection du mode de visualisation de densité osseuse par un signal de fonctionnement d'utilisateur.

10. Dispositif de traitement d'image (1) comprenant :
une unité de sortie (18) configurée pour superposer, par rapport à un dispositif de fixation posé (500) dans une image dentaire, une région d'affichage de densité osseuse virtuelle (510) sur une position du dispositif de fixation (500), et afficher une densité osseuse sous forme d'information de couleur dans la région d'affichage de densité osseuse (510), quand on entre un mode d'affichage de densité osseuse ;
une unité d'entrée (16) configurée pour recevoir un signal de fonctionnement d'utilisateur ; et
un contrôleur (14) configuré pour générer une région d'affichage de densité osseuse (510) tout en réalisant le mode d'affichage de densité osseuse en réponse au signal de fonctionnement d'utilisateur reçu, analyser une densité osseuse correspondant à une région d'affichage de densité osseuse préétablie, puis configurer et fournir, à l'unité de sortie (18), un écran pour afficher la densité osseuse analysée sous forme d'information de couleur dans la région d'affichage de densité osseuse (510),
dans lequel la région d'affichage de densité osseuse virtuelle (510) est placée à la position du dispositif de fixation (500) sous une forme transparente.

11. Dispositif de traitement d'image (1) selon la revendication 10, dans lequel :
l'unité de sortie (18) est en outre configurée pour placer la région d'affichage de densité osseuse (510) espacée d'un intervalle préétabli par rapport à une ligne de délimitation du dispositif de fixation (500) sur le dispositif de fixation dans un état transparent, et
l'intervalle préétabli de la région d'affichage de densité osseuse (510) peut être modifié par un utilisateur.

12. Dispositif de traitement d'image (1) selon la revendication 10, dans lequel le contrôleur (14) est configuré pour :
calculer une valeur de densité osseuse moyenne de la région d'affichage de densité osseuse (510) ; et
configurer un écran pour afficher la valeur de densité osseuse moyenne calculée sous la forme de ladite information de couleur.

13. Dispositif de traitement d'image (1) selon la revendication 10, dans lequel le contrôleur (14) est configuré pour :
classer un niveau de densité osseuse en os dur, os normal et os mou selon la qualité osseuse ; et
configurer un écran pour afficher la densité osseuse dans une couleur correspondant au niveau de densité osseuse déterminé dans la région d'affichage de densité osseuse (510).

14. Dispositif de traitement d'image (1) selon la revendication 10, dans lequel :
l'unité d'entrée (16) est configurée pour recevoir un signal de fonctionnement d'utilisateur pour entrer un mode d'affichage de densité osseuse multiple, et
le contrôleur (14) est configuré pour générer une pluralité d'images en coupe consécutives (610-1, 610-2, ..., 610-9) comportant une région d'affichage de densité osseuse préétablie en réponse au signal de fonctionnement d'utilisateur reçu, et fournir des coupes de densité osseuse consécutives en configurant un écran pour afficher la densité osseuse sous la forme de l'information de couleur dans la région d'affichage de densité osseuse (510) dans les images en coupe consécutives respectives (610-1, 610-2, ..., 610-9), quand le signal de fonctionnement d'utilisateur pour entrer le mode d'affichage de densité osseuse multiple est reçu par l'intermédiaire de l'unité d'entrée (16).

15. Dispositif de traitement d'image (1) selon la revendication 10, dans lequel :
l'unité d'entrée (16) est configurée pour recevoir un signal de fonctionnement d'utilisateur pour sélectionner une région de réglage d'utilisateur prédéterminée (810) pour identifier la densité osseuse dans l'image dentaire ; et
le contrôleur (14) est configuré pour analyser une densité osseuse correspondant à une région de réglage d'utilisateur (810) sélectionnée par un utilisateur en réponse au signal de fonctionnement d'utilisateur reçu et configurer un écran pour afficher la densité osseuse analysée sous la forme de l'information de couleur dans la région de réglage d'utilisateur (810).
